# EUROPEAN PATENT APPLICATION

(11) **EP 4 406 981 A1**
(43) Date of publication of application: **31.07.2024**
(21) Application number: 22872866.3
(22) Date of filing: 20.09.2022
(51) Int. Cl.: C08F 26/02, G01N 33/48

(54) **BLOOD CELL SEPARATING AGENT FOR ANALYSIS AND BLOOD CELL SEPARATION METHOD**

(30) Priority: 21.09.2021 JP 2021152920
(71) Applicant: Nitto Boseki Co., Ltd., Fukushima-shi Fukushima 960-8161 (JP)
(72) Inventor: MASUYA Amiko, Koriyama-shi, Fukushima 963-8061 (JP); YABE Kazuho, Koriyama-shi, Fukushima 963-8061 (JP); WATANABE Koji, Koriyama-shi, Fukushima 963-8061 (JP); TERUUCHI Yuya, Koriyama-shi, Fukushima 963-8061 (JP); TERUUCHI Yoko, Koriyama-shi, Fukushima 963-8061 (JP)
(74) Representative: Hoffmann Eitle
(86) International application number: PCT/JP2022/034889
(87) International publication number: WO 2023/048112

(57) **Abstract**

To provide a blood cell separating agent and a blood cell separation method capable of separating blood cells in a short time from a sample containing blood cells collected for analysis. The problem is solved by a blood cell separating agent for analysis that contains a (co)polymer having at least one structural unit of a structure derived from a monoallylamine or a diallylamine having a secondary amino group or a secondary amide group or a structure that is an inorganic acid salt or an organic acid salt thereof.

## Description

### Technical Field

The present invention relates to a blood cell separation agent and a blood cell separation method, and more particularly, it relates to a blood cell separation agent capable of rapidly separating a blood cell from a sample that is collected for analysis and contains a blood cell, and a blood cell separation method.

### Background Art

A plasma and a serum are widely used as analysis samples for various biochemical or immunological tests, and a centrifugation method is generally employed for separating a plasma or serum and a blood cell. Centrifugation requires, however, a comparatively long time, and hence a simpler and faster blood cell separation method not requiring centrifugation has been examined.

For example, for separating an erythrocyte from a blood sample, it has been proposed to agglomerate an erythrocyte with a solution containing a cholic acid-based surfactant and an acid (Patent Document 1).

A method for separating a serum and a clot by adding a specific quaternary amine sulfone polymer to collected blood has been proposed (Patent Document 2). This method requires, however, a blood clotting time of 30 minutes or more, and hence is insufficient in rapid blood cell separation.

In addition, inventions of a feminine hygiene product to which a cationic polymer that is a specific quaternary ammonium salt homopolymer or quaternary ammonium salt copolymer is applied have been proposed (Patent Documents 3 and 4). The inventions described in these documents improve, however, a menstrual blood absorbing speed in a feminine hygiene product by using the cationic polymer in the feminine hygiene product, and do no relate to a technique for separating a blood cell from a sample collected from a patient for analysis, diagnosis or the like.

### Prior Art Documents

### Patent Document

Patent Document 1: JP 2018-059791 A
Patent Document 2: JP 59-26062 A
Patent Document 3: JP 2019-10328 A
Patent Document 4: JP 2016-107100 A

### Summary of Invention

### Technical Problem

In consideration of the above-described conventional techniques, an object of the present invention is to provide an agent for use in blood cell separation and a method of separating blood cell, capable of separating, in a short time, a blood cell from a sample collected for analysis and containing a blood cell. In a preferable embodiment of the present invention, an object is to provide an agent for use in blood cell separation and a method of separating blood cell, capable of separating, in a short time, a blood cell from a sample collected for analysis and containing a blood cell, and capable of providing a sample less affecting analysis of a biochemical or immunological test.

### Solution to Problem

As a result of earnest studies, the present inventors have found that a blood cell can be separated in a short time, which cannot be achieved by conventional techniques, by using a (co)polymer having at least one constituent unit having a structure derived from a monoallylamine or diallylamine having a secondary amino group or a secondary amide group, or a structure of an inorganic acid salt or organic acid salt thereof, and thus, the present invention has been accomplished.

Specifically, the present invention relates to:
[1] An agent for use in blood cell separation for analysis, containing a (co)polymer having at least one constituent unit having a structure derived from a monoallylamine or diallylamine having a secondary amino group or a secondary amide group, or a structure of an inorganic acid salt or organic acid salt thereof.
   The following [2] to [17] are all preferable aspects or embodiments of the present invention:
[2] The agent for use in blood cell separation according to [1], wherein the (co)polymer is a diallylamine (co)polymer having at least one Diallylamine-based Constituent Unit (i) having a structure represented by the following structural formula (Ia) or (Ib), or a structure of an inorganic acid salt or organic acid salt thereof: 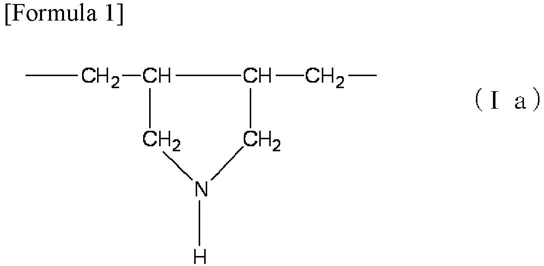 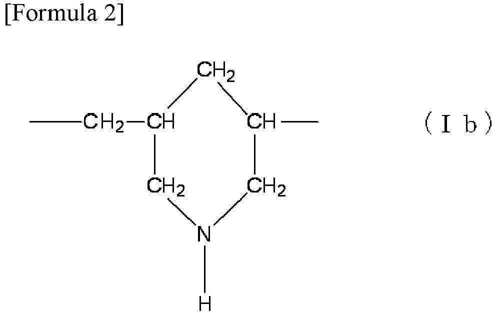
[3] The agent for use in blood cell separation according to [2], wherein Diallylamine-based Constituent Unit (i) has a structure of hydrochloride, carboxylate, sulfonate, or alkyl sulfate of the structure represented by the structural formula (Ia) or (Ib).
[4] The agent for use in blood cell separation according to any one of [1] to [3], wherein the (co)polymer further has at least one constituent unit selected from the group consisting of Anionic Constituent Unit (ii), Sulfur Dioxide Constituent Unit (iii), and (Di)allylamine Constituent Unit (iv) having neither a Secondary Amino Group nor a Secondary Amide Group.
[5] The agent for use in blood cell separation according to any one of [1] to [4], wherein the weight average molecular weight of the (co)polymer is 3,000 to 200,000.
[6] The agent for use in blood cell separation according to [4], wherein the (co)polymer has an Anionic Constituent Unit (ii), and the Anionic Constituent Unit (ii) is derived from a divalent carboxylic acid.
[7] The agent for use in blood cell separation according to any one of [1] to [6], wherein the (co)polymer is carried on an inorganic carrier.
[8] The agent for use in blood cell separation according to [7], wherein the (co)polymer is carried on the inorganic carrier in a state of being adsorbed through electrostatic interaction, and/or being bonded by a covalent bond.
[9] The agent for use in blood cell separation according to [8], wherein the covalent bond is made by silane coupling.
[10] A method for separating a blood cell, including the step of contacting a sample containing a blood cell with the agent for use in blood cell separation according to any one of [1] to [9].
[11] The method according to [10], including agglomerating the blood cell by mixing the sample containing a blood cell with the agent for use in blood cell separation according to any one of [1] to [9], and collecting a supernatant separated from the blood cell.
[12] The method according to [11], wherein the supernatant is collected 15 minutes after the mixing.
[13] The method according to any one of [10] to [12], wherein the sample is a whole blood specimen.
[14] A method for producing an agent for use in blood cell separation, including bonding, to an inorganic carrier, a (co)polymer having at least one constituent unit having a structure derived from a monoallylamine or diallylamine having a secondary amino group or a secondary amide group, or a structure of an inorganic acid salt or organic acid salt thereof.
[15] The method according to [14], wherein the (co)polymer is bonded to the inorganic carrier through electrostatic interaction.
[16] The method according to [14], wherein the (co)polymer is bonded to the inorganic carrier by a covalent bond.
[17] The method according to [16], including reacting the inorganic carrier, a silane coupling agent, and the (co)polymer for silane coupling the (co)polymer to the inorganic carrier.

### Advantageous Effects of Invention

An agent for use in blood cell separation and a method of separating blood cell of the present invention can separate a blood cell in a shorter time, and in addition, less affect biochemical or immunological analysis, and therefore, it is possible to provide a practically variable blood cell separation technique by which various analyses including general purpose automatic analysis, chromatography analysis such as HPLC, and point-of-care testing can be more rapidly conducted.

In a preferable embodiment, not only an erythrocyte but also another blood cell component can be separated. In addition, the agent for use in blood cell separation and the method of separating blood cell of the present invention less affect a larger number of items of the analysis, and are applicable to a larger number of tests.

### Brief Description of Drawing

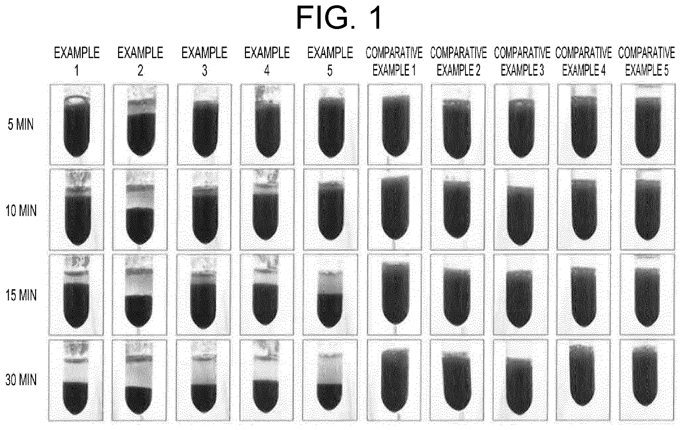

[Fig. 1] Fig. 1 illustrates photographs of a separation state between a blood cell and a supernatant obtained by adding 200 µL of a polymer solution of each of Examples 1 to 5 and Comparative Examples 1 to 5 to 1.0 mL of a whole blood specimen held in a microtube (made of PP) with a volume of 2.0 mL, and mixing the resultant by inversion, the separation state being checked 5 minutes, 10 minutes, 15 minutes, and 30 minutes after the mixing.

### Description of Embodiments

An agent for use in blood cell separation of the present invention contains a (co)polymer having at least one constituent unit having a structure derived from a monoallylamine or diallylamine having a secondary amino group or a secondary amide group, or a structure of an inorganic acid salt or organic acid salt thereof (hereinafter, also referred to as "Specific (Co)polymer").

The agent for use in blood cell separation of the present invention contains Specific (Co)polymer, and hence can realize excellent technical effects such as excellent blood cell separation performance.

The agent for use in blood cell separation of the present invention may contain only one type of Specific (Co)polymer, or may contain a combination of two or more types thereof.

### Specific (Co)polymer

The Specific (Co)polymer is only required to be a (co)polymer having at least one constituent unit having a structure derived from a monoallylamine or diallylamine having, in the molecule thereof, a secondary amino group or a secondary amide group, or a structure of an inorganic acid salt or organic acid salt thereof (hereinafter also referred to as the "Specific Constituent Unit"), and is not particularly limited otherwise.

A structure derived from a diallylamine does not have a secondary amide group, and hence, the "structure derived from a monoallylamine or diallylamine having, in the molecule thereof, a secondary amino group or a secondary amide group" can be expressed also as the "structure derived from a monoallylamine having, in the molecule thereof, a secondary amino group or a secondary amide group, or structure derived from a diallylamine having, in the molecule thereof, a secondary amino group".

Here, the term "(co)polymer" is a concept encompassing both a homopolymer and a copolymer, and accordingly, Specific (Co)polymer may be a homopolymer, which consists only of Specific Constituent Units, or may be a copolymer, which has another constituent unit(s) in addition to Specific Constituent Unit(s).

Specific (Co)polymer is only required to have only one Specific Constituent Unit in the molecule thereof, but from the viewpoints of blood cell separation performance and the like, it preferably has a plurality of Specific Constituent Units. More specifically, the ratio of Specific Constituent Units in all constituent units of Specific (Co)polymer is preferably 1 mol% or more, more preferably 10 to 100 mol%, and particularly preferably 50 to 100 mol%.

Specific (Co)polymer may have, in the molecule thereof, only one type of Specific Constituent Unit, or may have two or more types of Specific Constituent Units. When Specific Polymer has two or more types of Specific Constituent Units, the above-described ratio of Specific Constituent Units in all constituent units of the Specific (Co)polymer is calculated based on the total molar number of the two or more types of Specific Constituent Units.

### Diallylamine-based Constituent Unit (i)

Specific Constituent Unit may be a constituent unit having a structure derived from a monoallylamine having a secondary amino group or a secondary amide group, or a structure of an inorganic acid salt or organic acid salt thereof, or may be a constituent unit having a structure derived from a diallylamine having a secondary amino group, or a structure of an inorganic acid salt or organic acid salt thereof, and from the viewpoints of blood cell separation performance, influence on a biochemical or immunological analysis, and the like, it is preferably a constituent unit having a structure derived from a diallylamine having a secondary amino group, or a structure of an inorganic acid salt or organic acid salt thereof (hereinafter also referred to as "Diallylamine-based Constituent Unit (i)").

More specifically, Diallylamine-based Constituent Unit (i) has a structure represented by the following structural formula (Ia) or (Ib), or a structure of an addition salt (an inorganic acid salt or organic acid salt) thereof:

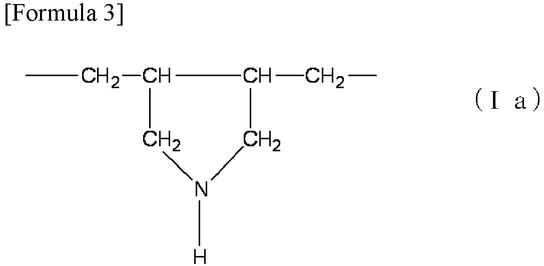

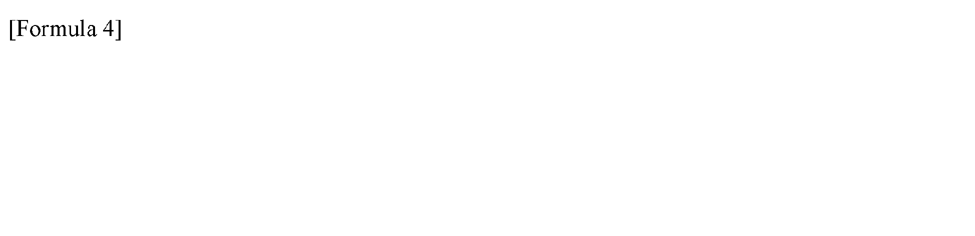

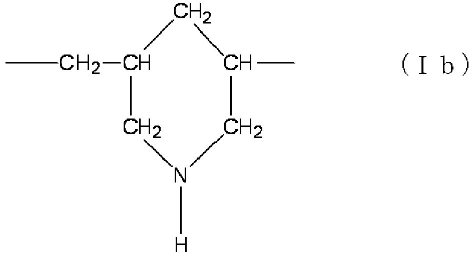

The structure of Diallylamine-based Constituent Unit (i) in the case of being an addition salt of the structure represented by the structural formula (Ia) or (Ib) is represented by the following structural formula (I'a) or (I'b), respectively. In these formulas, X is not particularly limited as long as it is a group capable of forming an inorganic acid or organic acid together with hydrogen.

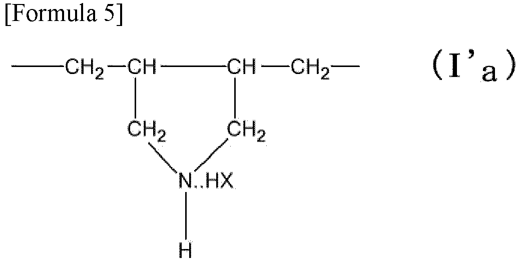

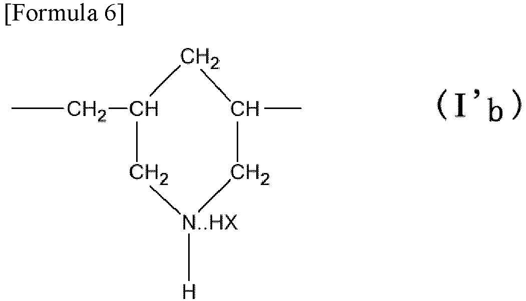

From the viewpoints of availability, reaction controllability and the like, the inorganic acid salt or organic acid salt of the structure represented by the structural formula (Ia) or (Ib) is preferably hydrochloride, carboxylate, sulfonate, or alkyl sulfate salt, and particularly preferably a hydrochloride.

In other words, in the structural formulas (I'a) and (I'b), X is preferably chlorine, a carboxylate group, a sulfonate group, or an alkyl sulfate group, and particularly preferably chlorine.

### Allylamine-based Constituent Unit

Another alternative of Specific Constituent Unit, that is, the constituent unit having a structure derived from a monoallylamine having a secondary amino group or a secondary amide group, or a structure of an inorganic acid salt or organic acid salt thereof, (hereinafter also referred to as "Monoallylamine-based Constituent Unit") more specifically has a structure represented by the following formula (II), or a structure of an addition salt thereof.

In formula (II), R¹ is an optionally substituted hydrocarbon group, the hydrocarbon group may have a hetero atom such as oxygen or sulfur as a result of substitution, and may be, for example, a substituted or unsubstituted acyl group. R¹ is preferably an alkyl group having 1 to 12 carbon atoms, a cycloalkyl group having 5 to 6 carbon atoms, or an acyl group having 2 to 5 carbon atoms.

An alkyl group having 1 to 12 carbon atoms, which is a preferable alternative of R¹, may be linear or branched, or may be an aralkyl group. Examples thereof include a methyl group, an ethyl group, a n-propyl group, an isopropyl group, a n-butyl group, an isobutyl group, a sec-butyl group, a tert-butyl group, a pentyl group, a hexyl group, an octyl group, a decyl group, a dodecyl group, and a benzyl group. Examples of a cycloalkyl group having 5 to 6 carbon atoms, which is another preferable alternative of R¹, include, but are not limited to, a cyclopentyl group, and a cyclohexyl group. Examples of an acyl group having 2 to 5 carbon atoms, which is another preferable alternative of R¹, include an unsubstituted acyl group, an acyl group substituted with alkoxy, and an acyl group substituted with amine, and more specific examples include, but are not limited to, a methylcarbonyl group, a methoxycarbonyl group, an aminocarbonyl group, an ethylcarbonyl group, a propylcarbonyl group, a butylcarbonyl group, an ethoxycarbonyl group, a propoxycarbonyl group, and butoxycarbonyl.

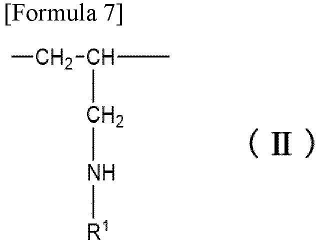

The structure of Monoallylamine-based Constituent Unit in the case of being an addition salt (an inorganic acid or organic acid) is represented by the following formula (II'):

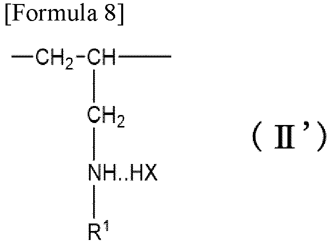

In formula (II'), HX represents an inorganic acid or an organic acid, H represents a hydrogen atom, and X is not especially limited as long as it is a group capable of forming an inorganic acid or an organic acid together with hydrogen.

The type of the addition salt is not particularly limited, and from the viewpoints of availability, reaction controllability, and the like, for example, hydrochloride, sulfate, phosphate, nitrate, sulfite, phosphite, nitrite, hydrobromide, acetate, amidosulfate, methanesulfonate, trifluoroacetate, and p-toluenesulfonate can be used.

In particular, hydrochloride, sulfate, phosphate, and amidosulfate are preferred, and hydrochloride, sulfate, phosphate, and amidosulfate having a structure derived from a monoallylamine are particularly preferred.

As described above, Specific (Co)polymer may have, in the molecule thereof, only one type of Specific Constituent unit, or may have two or more types of Specific Constituent Units. When Specific (Co)polymer has two or more types of Specific Constituent Units, the two or more types of Specific Constituent Units may be all Diallylamine-based Constituent Units (i), may be all Monoallylamine-based Constituent Units, or may be a combination of Diallylamine-based Constituent Unit(s) (i) and Monoallylamine-based Constituent Unit(s).

When Diallylamine-based Constituent unit(s) (i) and Monoallylamine-based Constituent Unit(s) are used in combination, the ratio between Diallylamine-based Constituent Unit(s) (i) and Monoallylamine-based Constituent Unit(s) is not particularly limited, and is preferably 100:1 to 1:100, and particularly preferably 10:1 to 1:10 in terms of a molar ratio.

### Other Constituent Unit(s)

When Specific (Co)polymer contains other constituent unit(s) in addition to Specific Constituent Unit(s), the constituent unit(s) used in addition to Specific Constituent Unit(s) is/are not particularly limited, and a monomer copolymerizable with a monoallylamine-based monomer or a diallylamine-based monomer can be appropriately used for introducing constituent unit(s) used in addition to Specific Constituent Unit(s).

Such a constituent unit is particularly preferably Anionic Constituent Unit (ii), Sulfur Dioxide Constituent Unit (iii), or (Di)allylamine-based Constituent Unit (iv) having neither a Secondary Amino Group nor a Secondary Amide Group, and at least one constituent unit selected from these can be introduced into Specific (Co)polymer. When such a constituent unit is introduced into Specific (Co)polymer in addition to Specific Constituent Unit(s), blood cell separation ability of the agent for use in blood cell separation can be improved, and influence on biochemical or immunological analysis can be reduced.

### Anionic Constituent Unit (ii)

In the present embodiment, when Specific (Co)polymer has Anionic Constituent Unit(s) (ii), the Specific (Co)polymer becomes what is called an amphoteric polymer, and thus, advantageous effects such as miscibility with a blood cell, urine or the like, and hydrophilicity can be realized. Anionic Constituent Unit (ii) is preferred also from the viewpoints of improving blood cell separation ability, and reducing influence on biochemical or immunological analysis.

The Anionic Constituent Unit(s) (ii) of the present embodiment of the invention is only required to be a constituent unit capable of having negative charge through desorption, and is not limited otherwise, and from the viewpoints of further improving blood cell separation ability, and further reducing influence on a biochemical or immunological analysis, preferably has a structure derived from a divalent carboxylic acid, and particularly preferably has a structure represented by the following structural formula (III), (IV), or (V):

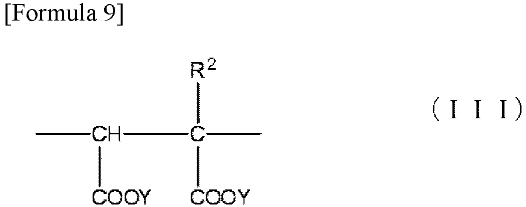

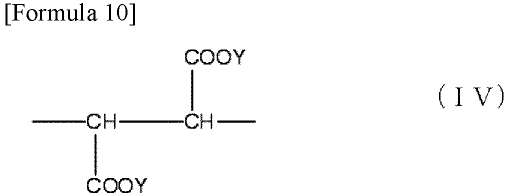

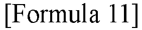

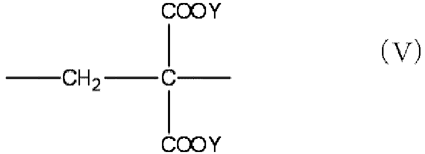

In the formula (III), R² is hydrogen or a methyl group, and in the formulas (III), (IV), and (V), Y is hydrogen, Na, K, NH₄, 1/2Ca, 1/2Mg, 1/2Fe, 1/3Al, or 1/3Fe respectively independently with respect to a carboxy group to be bonded.

Anionic Constituent Unit (ii) may have a structure corresponding to none of the structural formulas (III), (IV), and (V) as long as it has an anionic group such as a carboxyl group.

When Specific (Co)polymer has Anionic Constituent Unit(s) (ii), only one type of Anionic Constituent Unit (ii) may be singly used, or a plurality of types of Anionic Constituent Units (ii) having different structures may be used in combination.

When a plurality of types of Anionic Constituent Units (ii) different from one another are used, the respective Anionic Constituent Units may have structures different from one another as long as the structures are represented by the same general structural formula (III), (IV), or (V), or may have structures different from one another represented by different general structural formulas. In the former case, for example, a plurality of types of Anionic Constituent Units (ii) that all are represented by the general structural formula (III) but have different structures because the element of Y is different from one another may be used. In the latter case, one Anionic Constituent Unit (ii) having a structure represented by the structural formula (III), and another Anionic Constituent Unit (ii) having a structure represented by the structural formula (IV) may be used.

From the viewpoints of further improving blood cell separation ability, and further reducing influence on biochemical or immunological analysis, at least a part of Anionic Constituent Unit (ii) is preferably derived from maleic acid or fumaric acid, and in this case, the Anionic Constituent Unit (ii) has a structure represented by the formula (IV), in which Y is both hydrogen.

The content of Anionic Constituent Unit(s) (ii) when Specific (Co)polymer has Anionic Constituent Unit(s) (ii) is not especially limited, and when the Anionic Constituent Unit(s) (ii) is/are derived from an unsaturated dicarboxylic acid, the molar ratio to the Specific Constituent Unit(s) is preferably such that Specific Constituent Unit(s)/Anionic Constituent Unit(s) (ii) is 1/0.1 to 1/1, and particularly preferably 1/0.2 to 1/1.

At least a part of Anionic Constituent Unit(s) (ii) may be derived from an unsaturated monocarboxylic acid. When Anionic Constituent Unit(s) (ii) is derived from an unsaturated monocarboxylic acid, the ratio of Specific Constituent Unit(s)/Anionic Constituent Unit(s) (ii) is preferably 1/100 to 100/1, and particularly preferably 1/10 to 10/1.

### Sulfur Dioxide Constituent Unit (iii)

In the present embodiment, when Specific (Co)polymer has Sulfur Dioxide Constituent unit (iii), advantageous properties such as improvement of hydrophobicity, improvement of separation performance of an anionic component (such as a blood cell), and reduction of influence on biochemical or immunological analysis can be imparted to the Specific (Co)polymer.

Sulfur Dioxide Constituent unit (iii) in the (co)polymer of the present embodiment is derived from sulfur dioxide having a structure represented by the following structural formula (VI):

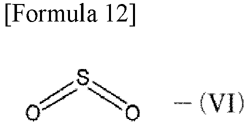

The ratio of Sulfur Dioxide Constituent Unit(s) (iii) in all constituent units of the (co)polymer of the present embodiment is preferably 1 mol% or more. In particular, when the ratio of Sulfur Dioxide Constituent Unit(s) (iii) is 1 mol% or more, advantageous properties such as improvement of hydrophobicity, improvement of separation performance of an anionic component (such as a blood cell), and reduction of influence on biochemical or immunological analysis can be more effectively imparted to the (co)polymer of the present embodiment.

The ratio of Sulfur Dioxide Constituent unit(s) (iii) is more preferably 1 to 50 mol%, and particularly preferably 10 to 50 mol%.

In the present embodiment, the ratio between Specific Constituent Unit(s) and Sulfur Dioxide Constituent Unit(s) (iii) is also not especially limited, and can be any ratio as long as these are copolymerizable. From the viewpoint of increasing the molecular weight of the copolymer, it is preferable that the ratios of these constituent units are not largely different from each other, and for example, the ratio between Specific Constituent Unit(s) (when a (Di)allylamine-based Constituent Unit(s) (iv) having neither a Secondary Amino Group nor a Secondary Amide Group is/are also contained, the sum of Specific Constituent Unit(s) and (Di)allylamine-based Constituent Unit(s) (iv) having neither a Secondary Amino Group nor a Secondary Amide Group) and Sulfur Dioxide Constituent Unit(s) (iii) is preferably 99:1 to 50:50, and particularly preferably 90:10 to 50:50.

### (Di)allylamine-based Constituent Unit (iv) having neither Secondary Amino Group nor Secondary Amide Group

In the present embodiment, when Specific (Co)polymer has (Di)allylamine-based Constituent Unit (iv) having neither a Secondary Amino Group nor a Secondary Amide Group, advantageous properties such as improvement of blood cell separation ability can be imparted to the Specific (Co)polymer.

(Di)allylamine-based Constituent Unit (iv) having neither a Secondary Amino group nor a Secondary Amide Group is a constituent unit having a structure derived from a monoallylamine or diallylamine, or a structure of an inorganic acid salt or organic acid salt thereof, and having, in the structure thereof, neither a secondary amino group nor a secondary amide group.

Only one type of (Di)allylamine-based Constituent Unit (iv) having neither a Secondary Amino Group nor a Secondary Amide Group may be used, or two or more types thereof may be used in combination.

When Specific (Co)polymer (Di)allylamine-based Constituent Unit (iv) having neither a Secondary Amino Group nor a Secondary Amide Group, the content of the Constituent Unit(s) (iv) is not especially limited, and the molar ratio to Specific Constituent Unit(s) is preferably such that Specific Constituent Unit(s)/Constituent Unit(s) (iv) is 1/100 to 100/1, and particularly preferably 1/10 to 10/1.

When (Di)allylamine-based Constituent Unit (iv) having neither a Secondary Amino Group nor a Secondary Amide Group is a constituent unit having a structure derived from a monoallylamine, or a structure of an inorganic acid salt or organic acid salt thereof, the Constituent Unit (iv) has, in the structure thereof, a primary amino group, and/or a tertiary amino group.

Preferable examples of such a Constituent Unit (iv) include constituent units derived from allylamine, allylamine hydrochloride, allylamine amide sulfate, allylamine acetate, dimethyl allylamine, dimethyl allylamine hydrochloride, dimethyl allylamine amide sulfate, dimethyl allylamine acetate and the like.

When (Di)allylamine-based Constituent Unit (iv) having neither a Secondary Amino Group nor a Secondary Amide Group is a constituent unit having a structure derived from a diallylamine, or a structure of an inorganic acid salt or organic acid salt thereof, the Constituent Unit (iv) has, in the structure thereof, a tertiary amino group and/or a quaternary amino group.

Preferable examples of such a Constituent Unit (iv) include constituent units derived from methyl diallylamine, methyl diallylamine hydrochloride, methyl diallylamine amide sulfate, methyl diallylamine acetate, diallyl dimethyl ammonium chloride, diallyl methyl ethyl ammonium ethyl sulfate and the like.

The molecular weight of Specific (Co)polymer is not especially limited, and Specific (Co)polymer having a favorable molecular weight according to a use form in the agent for use in blood cell separation, and relationship with another component or the like may be obtained or polymerized. From the viewpoints of blood cell separation ability, availability and the like, one having a weight average molecular weight (Mw) of 3,000 or more and 200,000 or less is usually used.

From the viewpoint of realizing excellent blood cell separation ability, the weight average molecular weight (Mw) of Specific (Co)polymer is preferably 100,000 or less, more preferably 50,000 or less, particularly preferably 20,000 or less, further preferably 15,000 or less, and still further preferably 5,000 or less.

From the viewpoint of conducting polymerization with practically allowable time and cost, the weight average molecular weight (Mw) of Specific (Co)polymer is preferably 1,000,000 or less, and more preferably 100,000 or less.

The weight average molecular weight (Mw) of Specific (Co)polymer can be measured, for example, with a liquid chromatograph by gel permeation chromatography (GPC method). More specifically, it can be measured by a method, for example, described in Examples of the present invention.

The molecular weight of Specific (Co)polymer can be appropriately adjusted by choosing the presence, or absence, type and composition of comonomer(s), the temperature, time and pressure employed in polymerization process, the type and amount of a radical initiator used in the polymerization process, and the like.

A rotational viscosity [η] of Specific (Co)polymer is also not especially limited, and can be appropriately set according to a use form in the agent for use in blood cell separation, and relationship with another component or the like, and is preferably 10 to 700 mPa·s (25°C), and particularly preferably 10 to 60 mPa·s (25°C).

The rotational viscosity [η] can be measured by a method usually employed in the art, and for example, can be measured with a digital B type viscometer DV-3T manufactured by AMETEK Brookfield. The measurement can be performed with a ULA adapter used, representatively with a liquid amount of 16 mL and a liquid temperature of 25°C.

Also, the rotational viscosity [η] can be appropriately adjusted by choosing a dilution concentration, the presence, or absence, type and composition of a comonomer, the temperature, time and pressure employed in the polymerization process, the type and amount of a radical initiator used in the polymerization process, and the like.

### Method for Producing Specific (Co)polymer

The method for producing Specific (Co)polymer is not especially limited, and Specific (Co)polymer can be produced by any methods conventionally known in this technical field, and can be produced by, for example, homopolymerizing a monoallylamine or diallylamine having a secondary amino group or a secondary amide group, or an inorganic acid salt or organic acid salt thereof, or copolymerizing it with another monomer if desired.

In homopolymerizing a monoallylamine or diallylamine having a secondary amino group or a secondary amide group, or an inorganic acid salt or organic acid salt thereof, or copolymerizing it with another monomer, a solvent is not especially limited, and may be an aqueous solvent, or an organic solvent such as alcohol, ether, sulfoxide, or amide, and is preferably an aqueous solvent.

In homopolymerizing a monoallylamine or diallylamine having a secondary amino group or a secondary amide group, or an inorganic acid salt or organic acid salt thereof, or copolymerizing it with another monomer, a monomer concentration is different depending on the type of the monomer, or the type of the solvent used in (co)polymerization, and in using an aqueous solvent, is usually 10 to 75% by weight. This (co)polymerization reaction is usually a radical polymerization reaction, and is performed in the presence of a radical polymerization catalyst. The type of the radical polymerization catalyst is not especially limited, and preferable examples thereof include peroxides such as t-butyl hydroperoxide, persulfates such as ammonium persulfate, sodium persulfate, and potassium persulfate, and azobis-based or diazo-based water-soluble azo compounds.

An amount of the radical polymerization catalyst to be added is generally 0.1 to 20 mol%, and preferably 1.0 to 10 mol% with respect to all monomers. A polymerization temperature is generally 0 to 100°C, and preferably 5 to 80°C, and a polymerization time is generally 1 to 150 hours, and preferably 5 to 100 hours. As a polymerization atmosphere, no serious problem occurs in polymerizability even in the air, and the polymerization can be performed in an inert gas atmosphere of nitrogen or the like.

### Agent for Use in Blood Cell Separation

The agent for use in blood cell separation of the present invention contains Specific (Co)polymer ((co)polymer having at least one constituent unit having a structure derived from a monoallylamine or diallylamine having a secondary amino group or secondary amide group, or a structure of an inorganic acid salt or organic acid salt thereof). Specific (Co)polymer may be directly used as an agent for use in blood cell separation, and is preferably used in the form of an aqueous solution by dissolving it in an aqueous solvent from the viewpoint of miscibility with a sample. As the aqueous solvent, pure water, saline and the like, which do not affect a sample and analysis, are preferred. In addition to Specific (Co)polymer, other blood cell separation components such as inorganic particles of glass, silica, kaolin, zeolite, bentonite, or the like, cholic acid, a quaternary ammonium salt homopolymer, a quaternary ammonium salt copolymer, and a quaternary amine sulfone polymer can be appropriately used in combination.

As described above, the agent for use in blood cell separation of the present invention may be composed of Specific (Co)polymer alone, and Specific (Co)polymer may be directly added to a sample containing a blood cell such as blood, but from the viewpoints of handleability, reuse of the agent for use in blood cell separation, and suppression of contamination of a sample separated from a blood cell with the agent for use in blood cell separation, an agent for use in blood cell separation in which Specific (Co)polymer is carried on an inorganic carrier may be used.

The shape of the inorganic carrier is not especially limited, and for example, a container, an inner wall of a tube, an inorganic particle, a magnetic bead, or the like can be used as the inorganic carrier. A material of the inorganic carrier can be glass, silica, kaolin, zeolite, bentonite or the like from a viewpoint that such a material itself has blood cell separation ability, and easily carries Specific (Co)polymer.

When a particulate inorganic carrier is used, the particle size of the inorganic carrier is also not especially limited, and a primary particle size is preferably 0.05 to 500 µm, and particularly preferably 0.1 to 50 µm.

In the present embodiment, a method for carrying Specific (Co)polymer on the inorganic carrier is not especially limited, and methods conventionally employed for carrying a polymer may be appropriately employed. It is particularly preferable to adsorb Specific (Co)polymer on the inorganic carrier through electrostatic interaction, to carry Specific (Co)polymer on the inorganic carrier by a covalent bond, and to employ a combination thereof.

A method for carrying Specific (Co)polymer on the inorganic carrier by a covalent bond is not also especially limited, and a silane coupling agent is preferably used, and more specifically, epoxy silane, chloro silane or the like is particularly preferably used.

### Method of Separating Blood Cell

When the agent for use in blood cell separation is contacted with a sample containing a blood cell, the blood cell is agglomerated, and thus, the blood cell can be separated from another component. Accordingly, a method of separating blood cell is not especially limited as long as the agent for use in blood cell separation is contacted with a blood cell, and a blood cell can be separated by various methods in accordance with the form of the agent for use in blood cell separation.

For example, when Specific (Co)polymer itself, or a solution of Specific (Co)polymer is used as the agent for use in blood cell separation, the agent for use in blood cell separation is added to and mixed with a sample containing a blood cell held in a collection container, or a sample containing a blood cell is added to and mixed in a collection container holding the agent for use in blood cell separation therein, and thus, the blood cell can be agglomerated and precipitated to separate the blood cell from another component. This method is one of the most versatile methods in clinical practice, and the agent for use in blood cell separation of the present invention exhibits excellent blood cell separation ability in this embodiment as verified in Examples described below. In this embodiment, the solution of Specific (Co)polymer is preferably a solution obtained by dissolving it in pure water. The Specific (Co)polymer itself, or the solution of the Specific (Co)polymer is added preferably in an amount of 1 µg to 500 µg of the Specific (Co)polymer per mL of a specimen, more preferably in an amount of 5 µg to 100 µg, and particularly preferably 5 µg to 20 µg.

The blood cell can be separated also by holding or impregnating Specific (Co)polymer itself, or a solution of Specific (Co)polymer in a filtering material such as filter paper, and adding a sample containing a blood cell thereto to collect the sample having passed through the filtering material. Alternatively, the blood cell can be separated also by mixing the agent for use in blood cell separation and a sample containing a blood cell, and adding the resultant mixture to a filtering material such as filter paper to collect the sample having passed through the filtering material. In these methods, it is preferable that the filtering material such as filter paper is filled in a container such as a column used as a support, and that the agent for use in blood cell separation or the mixture of the agent for use in blood cell separation and the sample is put thereto. In such a separation method, a separation speed can be controlled to some extent using a pump or the like, and the sample having passed through the container can be subjected to analysis, and therefore, the separation method is excellent in compatibility with HPLC, an automatic analyzer, and point-of-care testing.

When an agent for use in blood cell separation in which Specific (Co)polymer is carried on a particulate inorganic carrier is used, a blood cell can be separated from another component by the same method as that employed when Specific (Co)polymer itself or a solution of Specific (Co)polymer is used as the agent for use in blood cell separation. In the agent for use in blood cell separation in which Specific (Co)polymer is carried on a particulate inorganic carrier, blood cell separation ability can be imparted to the inorganic carrier itself by selecting a proper material of the inorganic carrier, and thus, the blood cell separation ability can be enhanced. Also in the agent for use in blood cell separation in which Specific (Co)polymer is carried on a particular inorganic carrier, when a filtering material such as filter paper is used as a support, or a container such as a column filled therewith is further used, a separation method excellent in compatibility with HPLC, an automatic analyzer, and point-of-care testing can be obtained.

When an agent for use in blood cell separation in which Specific (Co)polymer is carried on the inner wall surface of a sample collecting tube is used, a blood cell can be separated only by putting a sample into the collecting tube and mixing the resultant by inversion if necessary, and hence this method is convenient, and has a merit that the sample collecting tube in which the agent for use in blood cell separation is carried can be repeatedly used.

The agent for use in blood cell separation of the present invention has a high speed of agglomerating a blood cell, and hence can separate a blood cell within a time that cannot be achieved by a conventional agent for use in blood cell separation. Specifically, a blood cell can be separated from another component in less than 30 minutes, preferably 20 minutes or less, more preferably 15 minutes or less, and particularly preferably 10 minutes or less after causing the agent for use in blood cell separation to contact with or be mixed with a sample containing a blood cell. Accordingly, the present invention provides blood cell separation means adaptable to a test requiring rapid presentation of an analysis result such as point-of-care testing.

The method of the present invention is applicable to any sample containing a blood cell, and can be applied to, for example, blood, urine, lymph, a brain fluid, a body cavity fluid, an exudate fluid, digestive juices, and feces. A representative applicable sample is blood, which encompasses a whole blood specimen, and a blood specimen containing an anticoagulant such as heparin, and a whole blood specimen is the most representative sample.

A sample separated from a blood cell by the method of the present invention can be subjected to analysis as a specimen for various tests. As verified in Examples described below, a sample having been treated with Specific (Co)polymer less affects analysis results of a comparatively large number of test items of biochemical or immunological tests, and less affects analysis results of most of test items in using the Specific (Co)polymer of the preferable embodiment. Therefore, the method of the present invention can be employed in pretreatments of samples to be subjected to various tests.

### Examples

The present invention will now be more specifically described with reference to examples, and it is noted that the present invention is not limited to these.

### 1. Preparation of Agent for Use in Blood Cell Separation

Each of cationic polymers having a structure derived from a monoallylamine having a secondary amide group (Examples 1 and 2), cationic polymers having a structure derived from a diallylamine having a secondary amino group (Examples 3 to 5), and cationic polymers having a structure derived from a diallylamine or monoallylamine having a tertiary or quaternary amino group (Comparative Examples 1 to 5), all shown in the following tables, was dissolved in pure water to prepare a polymer solution in a polymer solid content concentration of 10% by weight, and the resultant was adjusted to pH 6.8 to 7.5 with 4 N NaOH. It is noted that the pH was measured with HORIBA desktop pH meter. This applies to the following Examples.

**[Table 1]**

| | Example 1 | Example 2 |
|---|---|---|
| Product Name | PAA-AC5000 (manufactured by Nittobo Medical Co., Ltd.) | PAA-N5000 (manufactured by Nittobo Medical Co., Ltd.) |
| Structural Formula | 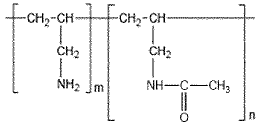 | 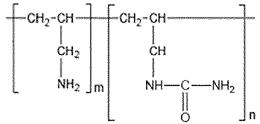 |
| Compound Name | Partially Methylcarbonylated Polyallylamine | Partially Created Polyallylamine |
| Weight Average Molecular Weight | 15,000 | 15,000 |
| Class of Amide | Secondary | Secondary |

**[Table 2]**

| | Example 3 | Example 4 | Example 5 |
|---|---|---|---|
| Product Name | PAA-D11-HCl (manufactured by Nittobo Medical Co., Ltd.) | PAS-92 (manufactured by Nittobo Medical Co., Ltd.) | P(DAA/FMA) (manufactured by Nittobo Medical Co., Ltd.) |
| Structural Formula | 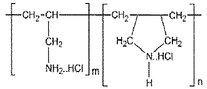 | 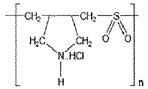 | 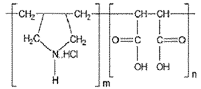 |
| Compound Name | Allylamine Hydrochloride-Diallylamine Hydrochloride Copolymer | Diallylamine Hydrochloride-Sulfur Dioxide Copolymer | Diallylamine Hydrochloride-Fumaric Acid Copolymer |
| Weight Average Molecular Weight | 100,000 | 5,000 | - |
| Class of Amine | Primary + Secondary | Secondary | Secondary |

**[Table 3]**

| | Comparative Example 1 | Comparative Example 2 |
|---|---|---|
| Product Name | PAS-2201CL (manufactured by Nittobo Medical Co., Ltd.) | PAS-A-5 (manufactured by Nittobo Medical Co., Ltd.) |
| Structural Formula | 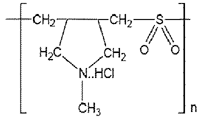 | 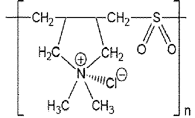 |
| Compound Name | Methyl Diallylamine Hydrochloride-Sulfur Dioxide Copolymer | Diallyl Dimethylammonium Chloride-Sulfur Dioxide Copolymer |
| Weight Average Molecular Weight | 3,000 | 4,000 |
| Class of Amine | Tertiary | Quaternary |

**[Table 4]**

| | Comparative Example 3 | Comparative Example 4 | Comparative Example 5 |
|---|---|---|---|
| Product Name | PAA-1112 (manufactured by Nittobo Medical Co., Ltd.) | PAS-2451 (manufactured by Nittobo Medical Co., Ltd.) | PAS-2351 (manufactured by Nittobo Medical Co., Ltd.) |
| Structural Formula | 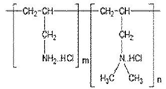 | 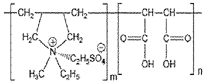 | 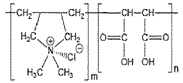 |
| Compound Name | Allylamine-Dimethyl Allylamine Copolymer | Diallyl Methylethyl Ammonium Ethylsulfate-Maleic Acid Copolymer | Diallyl Dimethylammonium Chloride-Maleic Acid Copolymer |
| Weight Average Molecular Weight | 1,000 | 30,000 | 25,000 |
| Class of Amine | Primary + Tertiary | Quaternary | Quaternary |

### (Method for Measuring Weight Average Molecular Weight)

The weight average molecular weight of each of the cationic polymers was measured by each of the following methods.

The weight average molecular weight (Mw) of a cationic polymer containing an Anionic Constituent Unit(s) (i) (Comparative Examples 4 and 5) was measured with Hitachi L-2000 High Performance Liquid Chromatograph by gel permeation chromatography (GPC method).

As an eluent flow path pump, Hitachi L-2130 pump was used, as a detector, Hitachi L-2490 RI detector was used, and as columns, two TOSOH TSKgel α-M (exclusion limit molecular weight: 10,000,000) connected in series were used. A sample was prepared to a concentration of 0.5 g/100 mL with an eluent, and was used in an amount of 100 µL. As the eluent, 0.15 mol/L sodium sulfate and 1 wt% acetic acid aqueous solution were used. The measurement was performed at a column temperature of 40°C at a flow rate of 1.0 mL/min. A calibration curve was obtained by using, as a standard substance, pullulan having a molecular weight of 5,900, 47,300, 212,000, and 788,000, and the weight average molecular weight (Mw) of the cationic polymer was obtained based on the calibration curve.

A weight average molecular weight (Mw) of other cationic polymers was measured with Chromaster(R) 5450 High Performance Liquid Chromatograph manufactured by Hitachi High-Tech Science Corporation by gel permeation chromatography (GPC method).

As a detector, an RI refractive index detector was used, and as columns, aqueous gel filtration type columns GS-220HQ (exclusion limit molecular weight: 3,000) and GS-620HQ (exclusion limit molecular weight: 2,000,000) manufactured by Shodex Asahipak connected in series were used. A sample was prepared to a concentration of 0.5 g/100 mL with an eluent, and was used in an amount of 20 µL. As the eluent, a 0.4 mol/L sodium chloride aqueous solution was used. The measurement was performed at a column temperature of 30°C at a flow rate of 1.0 mL/min. A calibration curve was obtained by using, as a standard substance, polyethylene glycol having a molecular weight of 106, 194, 440, 600, 1,470, 4,100, 7,100, 10,300, 12,600, 23,000 and the like, and the weight average molecular weight (Mw) of the cationic polymer was obtained based on the calibration curve.

### 2. Removal of Blood Cell with Polymer Solution

To 1.0 mL of a whole blood specimen held in a microtube (made of PP) with a volume of 2.0 mL, 200 µL of each polymer solution was added, and the resultant was mixed by inversion to test whether or not blood cells can be separated by agglomeration of blood cells. A sample obtained by adding saline instead of the polymer solution and mixing the resultant by inversion was used as a control.

Blood cell separation ability of each polymer was evaluated based on calculation of the number of blood cells and appearance observation.

### (1) Calculation of Number of Blood Cells

A supernatant (an aliquot in the vicinity of the surface when the blood was not agglomerated) was collected 15 minutes and 30 minutes after the mixing by inversion, and the numbers of leukocytes, erythrocytes, and platelets therein were measured with a blood cell analyzer (XS-500i manufactured by Sysmex Corporation). The thus measured numbers of blood cells were compared with the numbers of blood cells in the control to be determined in accordance with the following criteria:

### (Evaluation Criteria)

○: The number of blood cell components in the supernatant was reduced to less than 20% as compared with that in the control within 15 minutes after the mixing by inversion.
△: The number of blood cell components in the supernatant was reduced to less than 30% as compared with that in the control within 30 minutes after the mixing by inversion.
×: The number of blood cell components in the supernatant was 30% or more as compared with that in the control 30 minutes after the mixing by inversion.

### (2) Appearance Observation

A separation state between the blood cell and the supernatant was observed 5 minutes, 10 minutes, 15 minutes, and 30 minutes after the mixing by inversion.

Test results are shown in the following Tables 5 and 6, and in Fig. 1.

**[Table 5]**

| | Example 1 | Example 2 | Example 3 | Example 4 | Example 5 |
|---|---|---|---|---|---|
| Product Name | PAA-AC5000 | PAA-N5000 | PAA-D11-HCl | PAS-92 | P (DAA/FMA) |
| Structural Type | Secondary Amide-based | | Secondary Diallylamine-based | | |
| Leukocytes | ○ | × | × | ○ | ○ |
| Erythrocytes | ○ | ○ | ○ | ○ | ○ |
| Platelets | ○ | ○ | ○ | ○ | ○ |

**[Table 6]**

| | Comparative Example 1 | Comparative Example 2 | Comparative Example 3 | Comparative Example 4 | Comparative Example 5 |
|---|---|---|---|---|---|
| Product Name | PAS-2201CL | PAS-A-5 | PAA-1112 | PAS-2451 | PAS-2351 |
| Structural Type | Tertiary or Quaternary Diallylamine-based | | Allylamine-dimethyl Allylamine Copolymer | Quaternary Ammonium Salt Amphoteric-based | |
| Leukocytes | × | × | Δ | × | × |
| Erythrocytes | × | × | × | × | × |
| Platelets | × | × | × | × | × |

As illustrated in Fig. 1, when the cationic polymers having structures derived from a monoallylamine having a secondary amide group, that is, PAA-AC5000 and PAA-N5000, and the cationic polymers having a structure derived from a diallylamine having a secondary amino group, that is, PAA-D11-HC1, PAS-92 and P(DAA/FMA), were added to a whole blood specimen, separation between a plasma and a blood cell was visually confirmed within 10 minutes at the latest. As shown in Table 5, the number of erythrocytes in the supernatants of these specimens were all reduced to less than 20% as compared with that in the control in 15 minutes after the mixing by inversion. When PAA-AC5000, PAA-N5000, PAA-D11-HC1, PAS-92, and P(DAA/FMA) were added to a whole blood specimen, the numbers of erythrocytes and platelets in the supernatants were reduced to less than 20% as compared with those in the control in 15 minutes after the mixing by inversion, and when PAA-AC5000, PAS-92, and P(DAA/FMA) were added to a whole blood specimen, the numbers of leukocytes, erythrocytes, and platelets in the supernatants were reduced to less than 20% as compared with those in the control in 15 minutes after mixing by inversion.

On the other hand, as shown in Table 6 and Fig. 1, when the cationic polymers having a structure derived from a diallylamine or monoallylamine having a tertiary or quaternary amino group, that is, PAS-2201CL (methyl diallylamine hydrochloride sulfur dioxide copolymer), PAS-A-5 (diallyl dimethyl ammonium chloride sulfur dioxide copolymer), PAA-1112 (allylamine dimethyl allylamine copolymer), PAS-2451 (diallyl methylethyl ammonium ethylsulfate maleic acid copolymer), or PAS-2351 (diallyl dimethylammonium chloride maleic acid copolymer) was added to a whole blood specimen, blood cells did not precipitate in any specimen, and a plasma could not be captured.

### 3. Influence on Biochemical and Immunological Tests of Plasma Separated by Each Polymer

A whole blood specimen having a pseudo high value was prepared by adding, to 1.0 mL of a whole blood specimen held in a microtube with a volume of 2.0 mL, 100 µL of Aalto Control Level IIα (manufactured by Shino-Test Corporation) dissolved in pure water in an amount half the regulated amount and 15 µL of a concentration of 100 mg/dL CRP antigen, and biochemical and immunological tests were conducted using this specimen. To 1.0 mL of the whole blood specimen having a pseudo high value held in a microtube with a volume of 2.0 mL, 200 µL of each polymer solution was added, and the supernatant was collected 15 minutes after mixing the resultant by inversion. The thus collected supernatant was used for performing measurement of 10 biochemical test items and 3 immunological test items with Hitachi Automatic Analyzer 7180 (manufactured by Hitachi, Ltd.). As a control, a plasma obtained by centrifugation at 3,000 rpm for 10 minutes of blood collected in a plasma collecting tube with heparin (manufactured by Terumo Corporation) was used, and 200 µL of saline was added to 1.0 mL of the obtained plasma for making the dilution ratio the same as that of the blood sample diluted with the polymer solution.

Reagents used in the measurement of the 10 biochemical test items and the 3 immunological test items were as follows:
Reagents:
AST: N-assay LAST Nittobo (manufactured by Nittobo Medical Co., Ltd.)
ALT: N-assay L ALT Nittobo (manufactured by Nittobo Medical Co., Ltd.)
LDH: N-assay L LDH Nittobo B-Type (manufactured by Nittobo Medical Co., Ltd.)
γ-GTP: N-assay L γ-GTP-H Nittobo B-Type (manufactured by Nittobo Medical Co., Ltd.)
ALB: N-assay L ALB-S Nittobo (manufactured by Nittobo Medical Co., Ltd.)
CRE: N-assay L CRE-K Nittobo (manufactured by Nittobo Medical Co., Ltd.)
LDL: Metabolead LDL-C (manufactured by Hitachi Chemical Diagnostics Systems Co., Ltd.)
HDL: Metabolead HDL-C (manufactured by Hitachi Chemical Diagnostics Systems Co., Ltd.)
TCHO: N-assay L T-CHO-H Nittobo (manufactured by Nittobo Medical Co., Ltd.)
TG: Determiner L TG II (manufactured by Hitachi Chemical Diagnostics Systems Co., Ltd.)
CRP: N-assay LA CRP-S Nittobo D-Type (manufactured by Nittobo Medical Co., Ltd.)
IgG: N-assay TIA IgG-SH Nittobo (manufactured by Nittobo Medical Co., Ltd.)
C3: N-assay TIA C3-SH Nittobo (manufactured by Nittobo Medical Co., Ltd.)

For calibration, Aalto Control Level IIα (manufactured by Shino-Test Corporation) was used for the 10 biochemical test items, and a calibrator specified by the manufacturer of the reagent was used for the 3 immunological test items. Parameters of the respective tests were set according to instructions attached to the kits.

Test results of the respective test items of the specimens resulting from treatment with the respective polymers are all shown in the following. It is noted that each numerical value except a control measured value is shown as a relative value obtained assuming that the control measured value is 100.

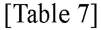

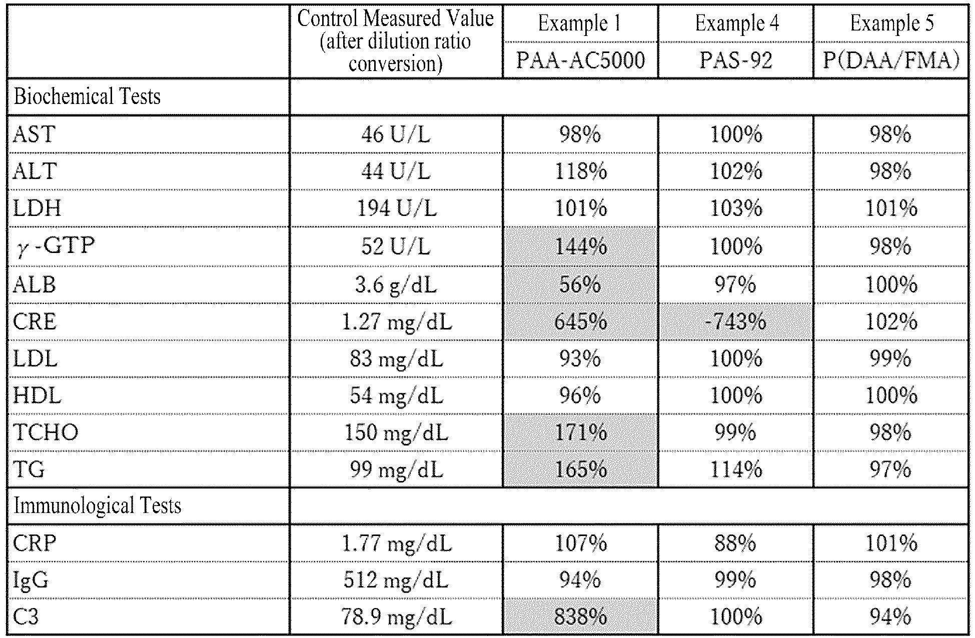

As shown above, in the specimen resulting from the treatment with PAA-AC5000, that is, the cationic polymer having a structure derived from a monoallylamine having a secondary amide group, the measurement could be conducted within an error margin of ±20% or less for 5 items out of the 10 biochemical test items, and 2 items out of the 3 immunological test items. On the contrary, in the plasmas obtained by treating a whole blood specimen with PAS-92 and P(DAA/FMA), that is, the cationic polymers having a structure derived from a diallylamine having a secondary amino group, it was revealed that the measurement can be conducted within an error margin of ±20% or less for 9 items out of the 10 biochemical test items, and all the 3 immunological test items.

### Industrial Applicability

An agent for use in blood cell separation, and a method of separating blood cell and the like using the same of the present invention can separate a blood cell in a shorter time, and in addition, less affect biochemical or immunological analysis, and therefore, are practically valuable, for example, can make it possible to more rapidly perform various analyses including general purpose automatic analysis, chromatography analysis such as HPLC, and point-of-care testing, and thus are highly applicable in industrial fields of analytical equipment and the like.

## Claims

1. An agent for use in blood cell separation for analysis, comprising a (co)polymer having at least one constituent unit having a structure derived from a monoallylamine or diallylamine having a secondary amino group or a secondary amide group, or a structure of an inorganic acid salt or organic acid salt thereof.

2. The agent for use in blood cell separation according to claim 1,
wherein the (co)polymer is a diallylamine (co)polymer having at least one Diallylamine-based Constituent Unit (i) having a structure represented by the following structural formula (Ia) or (Ib), or a structure of an inorganic acid salt or organic acid salt thereof:

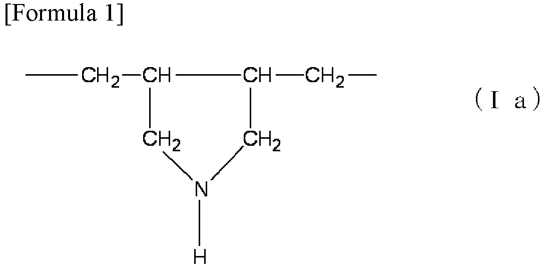

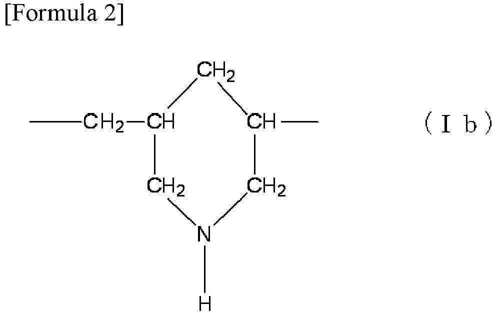

3. The agent for use in blood cell separation according to claim 2, wherein Diallylamine-based Constituent Unit (i) has a structure of hydrochloride, carboxylate, sulfonate, or alkyl sulfate of the structure represented by the structural formula (Ia) or (Ib).

4. The agent for use in blood cell separation according to any one of claims 1 to 3, wherein the (co)polymer further has at least one constituent unit selected from the group consisting of Anionic Constituent Unit (ii), Sulfur Dioxide Constituent Unit (iii), and (Di)allylamine Constituent Unit (iv) having neither a secondary amino group nor a secondary amide group.

5. The agent for use in blood cell separation according to any one of claims 1 to 4, wherein the weight average molecular weight of the (co)polymer is 3,000 to 200,000.

6. The agent for use in blood cell separation according to claim 4, wherein the (co)polymer has Anionic Constituent Unit (ii), and the Anionic Constituent Unit (ii) is derived from a divalent carboxylic acid.

7. The agent for use in blood cell separation according to any one of claims 1 to 6, wherein the (co)polymer is carried on an inorganic carrier.

8. The agent for use in blood cell separation according to claim 7, wherein the (co)polymer is carried on the inorganic carrier in a state of being adsorbed through electrostatic interaction, and/or being bonded by a covalent bond.

9. The agent for use in blood cell separation according to claim 8, wherein the covalent bond is made by silane coupling.

10. A method for separating a blood cell, comprising the step of contacting a sample containing a blood cell with the agent for use in blood cell separation according to any one of claims 1 to 9.

11. The method according to claim 10, comprising agglomerating the blood cell by mixing the sample containing a blood cell with the agent for use in blood cell separation according to any one of claims 1 to 9, and collecting a supernatant separated from the blood cell.

12. The method according to claim 11, wherein the supernatant is collected 15 minutes after the mixing.

13. The method according to any one of claims 10 to 12, wherein the sample is a whole blood specimen.

14. A method for producing an agent for use in blood cell separation, comprising bonding, to an inorganic carrier, a (co)polymer having at least one constituent unit having a structure derived from a monoallylamine or diallylamine having a secondary amino group or a secondary amide group, or a structure of an inorganic acid salt or organic acid salt thereof.

15. The method according to claim 14, wherein the (co)polymer is bonded to the inorganic carrier through electrostatic interaction.

16. The method according to claim 14, wherein the (co)polymer is bonded to the inorganic carrier by a covalent bond.

17. The method according to claim 16, comprising reacting the inorganic carrier, a silane coupling agent, and the (co)polymer for silane coupling the (co)polymer to the inorganic carrier.
